# EUROPEAN PATENT SPECIFICATION

(11) **EP 2 209 389 B1**
(45) Date of publication and mention of the grant of the patent: **28.12.2011**
(21) Application number: 08805128.9
(22) Date of filing: 08.10.2008
(51) Int. Cl.: A23L 1/03, C11B 9/00, C07C 33/12, C07D 307/93

(54) **BICYCLIC CAMPHOLENIC DERIVATIVES**
BICYCLISCHE CAMPHOLENDERIVATE
DERIVES CAMPHOLENIQUES BICYCLIQUES

(30) Priority: 09.10.2007 EP 07118147; 09.10.2007 US 978465 P
(43) Date of publication of application: 28.07.2010
(73) Proprietor: V. Mane Fils, 06620 Bar sur Loup (FR)
(72) Inventor: MANE, Jean, 06130 Grasse (FR); PLESSIS, Caroline, 06740 Chateauneuf (FR)
(74) Representative: Noel, Chantal Odile
(86) International application number: PCT/EP2008/063433
(87) International publication number: WO 2009/047258

(56) References cited:
- US-A- 5 276 211
- US-A- 5 510 326
- J.M. CASTRO ET AL: "3-Methyl-3-(6,6,6a-trimethyl-hexahydro-cy clopenta[b]furan-2-yl)-butan-2-one" MOLBANK, [Online] 1 July 2005 (2005-07-01), XP002475256 Retrieved from the Internet: URL:http://www.mdpi.net/molbank/molbank200 5/m393.htm> [retrieved on 2008-04-02]
- A. BÉHAL ET AL.: "Sur un isomère du bornéol, l'alcool campholénique et quelques dérivés campholéniques." BULL. SOC. CHIM. FR., vol. 13, no. 3, 1904, pages 179-185, XP009098083
- C. CHAPUIS ET AL.: "Anaolgues of alpha-campholenal as building blocks for (+)-beta-necrodol and sandalwood-like alcohols." HELV. CHIM. ACTA, vol. 89, no. 11, 2006, pages 2638-2653, XP002475255
- A. P.S. NARULA: "The search for new fragrance ingredients." PERFUMER & FLAVOURIST, 2003, pages 62-73, XP009098068 cited in the application

## Description

### [FIELD OF THE INVENTION]

The present invention relates to the field of fragrances. More particularly, the invention relates to new campholenic derivatives, their method of preparation, and their use in the fields of perfumery.

### [BACKGROUND]

Several bicyclic ethers derived from campholenaldehyde, substituted with a phenyl group or a phenyl substituted C₁-C₂ alkyl group, presenting a sweaty, animal, ambery (or woody) odour, have been described in the literature, e.g. in US patent n° 5,276,211.

Bicyclic ethers derived from campholenaldehyde, substituted with an ethyl, phenyl, benzyl, *n*-propyl, or ethyl-phenyl group in 2-position have been mentioned in an article (Narula, Perfumer & Flavourist, 2003, p. 62 to 73). To the best of our knowledge no other alkyl or alkenyl substituted bicyclic ether derivatives of campholenaldehyde have been described nor claimed to be good odorants. Furthermore, all the processes for preparing bicyclic ethers derived from campholenaldehyde described in the literature lead to several derivatives and the bicyclic ethers are obtained as a mixture of isomers. To the best of our knowledge, no processes allowing the selective synthesis of one bicyclic isomer have been described.

### [PROBLEM TO BE SOLVED]

In view of the drawbacks of the prior art, the Applicant focused on a process to prepare new alkyl or alkenyl substituted bicyclic ethers derived from campholenaldehyde.

The need for new compounds is of great importance for the development of the fragrance industry, which recently had to face stricter international regulatory requirements about the use of certain materials, as well as environmental concerns and customer demands for improved performance. Providing new fragrant compounds as well as means of selectively manufacturing such compounds, in particular as a single derivative, is therefore an object of the invention.

In other words, it is an aim of the present invention to provide new fragrant compounds, as well as a method of manufacturing such compounds.

### [SUMMARY OF THE INVENTION]

The present invention is directed to novel compounds of formula (I) 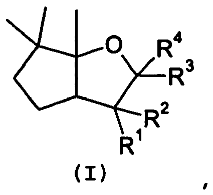 wherein
R¹, R², R³, and R⁴ are, independently, a hydrogen atom or an optionally substituted linear or branched C₁-C₆ alkyl or C₂-C₆ alkenyl group,
provided that when each of R¹, R², and R³ are a hydrogen atom, R⁴ is not a phenyl substituted linear or branched C₁-C₆ alkyl or C₂-C₆ alkenyl group; and
with the proviso that the compound of formula (I) is neither 6,6,6a-trimethyl-hexahydro-cyclopenta[b]furan, 6,6,6a-trimethyl-2-ethyl-hexahydro-cyclopenta[b]furan, 6,6,6a-trimethyl-2-propyl-hexahydro-cyclopenta[b]furan, nor 3-methyl-3-(6,6,6a-trimethyl-hexahydro-cyclopenta[b]furan-2-yl)-butanone.

The invention is also directed to a method of preparation of compounds of formula (I): 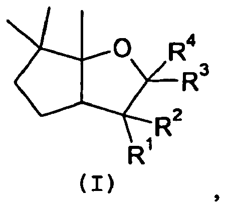 wherein
R¹, R², R³, and R⁴ are, independently, a hydrogen atom or an optionally substituted linear or branched C₁-C₆ alkyl or C₂-C₆ alkenyl group; and to intermediates or side products obtained thereby.

### [DETAILED DESCRIPTION OF THE INVENTION]

As noted above, the invention relates to compounds of formula (I), wherein R¹, R², R³, and R⁴ are, independently, a hydrogen atom or an optionally substituted linear or branched C₁-C₆ alkyl or C₂-C₆ alkenyl group,
provided that when each of R¹, R², and R³ are a hydrogen atom, R⁴ is not a phenyl substituted linear or branched C₁-C₆ alkyl or C₂-C₆ alkenyl group; and
with the proviso that the compound of formula (I) is neither 6,6,6a-trimethyl-hexahydro-cyclopenta[b]furan, 6,6,6a-trimethyl-2-ethyl-hexahydro-cyclopenta[b]furan, 6,6,6a-trimethyl-2-propyl-hexahydro-cyclopenta[b]furan, nor 3-methyl-3-(6,6,6a-trimethyl-hexahydro-cyclopenta[b]furan-2-yl)-butanone.

Suitable substituents for the alkyl or alkenyl groups comprise hydroxyl, amino groups, aryl groups, such as phenyl, tolyl (e.g. *p*-tolyl or o-tolyl), *p*-methoxy-phenyl, heterocycloalkyl groups, such as pyrolidinyl, tetrahydro-furanyl, and tetrahydro-pyranyl, heteroaryl groups such as pyridinyl, furanyl, and pyranyl, O-alkyl groups, such as methoxy, ethoxy, *i*-propoxy, phenoxy.

Preferred compounds of formula (I) are those, wherein R¹, R², R³, and R⁴ are, independently, a hydrogen atom or an optionally substituted methyl, ethyl, *n*-propyl, *i-*propyl, *n*-butyl, *i*-butyl, *s*-butyl, *t*-butyl, *n*-pentyl, *i*-pentyl, *t*-amyl, 1-ethyl-propyl, 1,2-dimethyl-propyl, 2,2-dimethyl-propyl, *n*-hexyl, 1-methyl-pentyl group, 2-methyl-pentyl group, 3-methyl-pentyl group, 4-methyl-pentyl group, 1,1-dimethyl-butyl, 1,2-dimethyl-butyl, 1,3-dimethyl-butyl, 2,2-dimethyl-butyl, 3,3-dimethyl-butyl, 1-ethyl-butyl, 2-ethyl-butyl, 1,1,2-trimethyl-propyl, 1,2,2-trimethyl-propyl, 1-ethyl-1-methyl-propyl or 1-ethyl-2-methyl-propyl, or an optionally substituted vinyl, 1-propenyl, 1*-i*-propenyl, 2-propenyl, 2-methyl-1-propenyl, 1-butenyl, 2-butenyl, 3-butenyl, 1-pentenyl, 2-pentenyl, 1-hexenyl, 2-hexenyl, 3-hexenyl, 2,4-pentadienyl group, with, as mentioned above, the proviso that when each of R¹, R², and R³ are a hydrogen atom, R⁴ is not a phenyl substituted linear or branched C₁-C₆ alkyl or C₂-C₆ alkenyl group. More preferably, R¹, R², R³, and R⁴ are, independently, a hydrogen atom or an optionally substituted methyl, ethyl, *n*-propyl, *i-*propyl, *n*-butyl, *i*-butyl, *s*-butyl, *t*-butyl, vinyl or propenyl group, with the proviso that when each of R¹, R², and R³ are a hydrogen atom, R⁴ is not phenyl substituted. Even more preferably, R¹, R², R³, and R⁴ are, independently, a hydrogen atom or an optionally substituted ethyl, *n*-butyl, *i*-butyl, *s*-butyl, *t*-butyl, or vinyl group, with the proviso that when each of R¹, R², and R³ are a hydrogen atom, R⁴ is not phenyl substituted.

Particularly preferred compounds of formula (I) are those, wherein R¹, R², and R³, are each a hydrogen atom and R⁴ is an optionally substituted linear or branched C₁-C₆ alkyl or C₂-C₆ alkenyl group, such as an optionally substituted methyl, ethyl, *n*-propyl, *i*-propyl, *n*-butyl, *i*-butyl, *s*-butyl, *t*-butyl, *n*-pentyl, *i*-pentyl, *t*-amyl, 1-ethyl-propyl, 1,2-dimethyl-propyl, 2,2-dimethyl-propyl, *n*-hexyl, 1-methyl-pentyl group, 2-methyl-pentyl group, 3-methyl-pentyl group, 4-methyl-pentyl group, 1,1-dimethyl-butyl, 1,2-dimethyl-butyl, 1,3-dimethyl-butyl, 2,2-dimethyl-butyl, 3,3-dimethyl-butyl, 1-ethyl-butyl, 2-ethyl-butyl, 1,1,2-trimethyl-propyl, 1,2,2-trimethyl-propyl, 1-ethyl-1-methyl-propyl or 1-ethyl-2-methylpropyl, or an optionally substituted vinyl, 1-propenyl, 1-i-propenyl, 2-propenyl, 2-methyl-1-propenyl, 1-butenyl, 2-butenyl, 3-butenyl, 1-pentenyl, 2-pentenyl, 1-hexenyl, 2-hexenyl, 3-hexenyl, 2,4-pentadienyl group, with the proviso that the optional substituent is not a phenyl group. More preferably, R¹, R², and R³, are each a hydrogen atom and R⁴ is an optionally substituted methyl, ethyl, *n*-propyl, *i*-propyl, *n*-butyl, *i*-butyl, *s*-butyl, *t*-butyl, vinyl or propenyl group, with the proviso that R⁴ is not phenyl substituted. Even more preferably, R¹, R², R³ are each a hydrogen atom and R⁴ is an optionally substituted ethyl, *n*-butyl, *i*-butyl, *s*-butyl, *t*-butyl, or vinyl group, with the proviso that R⁴ is not phenyl substituted.

The invention is also directed to a method of preparing a compound of general formula (I) 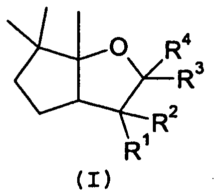 wherein
R¹, R², R³, and R⁴ are, independently, a hydrogen atom or an optionally substituted linear or branched C₁-C₆ alkyl or C₂-C₆ alkenyl group,
the method comprising reacting a compound of formula (III) 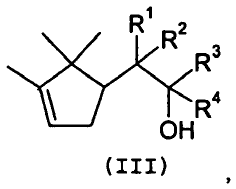 wherein
R¹, R², R³, and R⁴ are defined as in respect of formula (I),
with phosphoric acid, preferably concentrated phosphoric acid, in an organic solvent so as to obtain a mixture comprising a compound of formula (I) and a compound of formula (II) 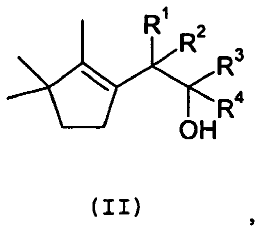 wherein
R¹, R², R³, and R⁴ are defined as in respect of formula (I).

Suitable substituents for the alkyl or alkenyl groups are those listed above in respect of compounds of formula (I).

Preferred compounds of formula (I) obtained according to the method of the invention are those, wherein R¹, R², R³, and R⁴ are, independently, a hydrogen atom or an optionally substituted methyl, ethyl, *n*-propyl, *i*-propyl, *n*-butyl, *i*-butyl, *s*-butyl, *t*-butyl, *n-*pentyl, *i*-pentyl, *t*-amyl, 1-ethyl-propyl, 1,2-dimethyl-propyl, 2,2-dimethyl-propyl, *n*-hexyl, 1-methyl-pentyl group, 2-methyl-pentyl group, 3-methyl-pentyl group, 4-methyl-pentyl group, 1,1-dimethyl-butyl, 1,2-dimethyl-butyl, 1,3-dimethyl-butyl, 2,2-dimethyl-butyl, 3,3-dimethyl-butyl, 1-ethyl-butyl, 2-ethyl-butyl, 1,1,2-trimethyl-propyl, 1,2,2-trimethyl-propyl, 1-ethyl-1-methyl-propyl or 1-ethyl-2-methyl-propyl, or an optionally substituted vinyl, 1-propenyl, 1-*i*-propenyl, 2-propenyl, 2-methyl-1-propenyl, 1-butenyl, 2-butenyl, 3-butenyl, 1-pentenyl, 2-pentenyl, 1-hexenyl, 2-hexenyl, 3-hexenyl, 2,4-pentadienyl group. More preferably, R¹, R², R³, and R⁴ are, independently, a hydrogen atom or an optionally substituted methyl, ethyl, *n*-propyl, *i*-propyl, *n*-butyl, *i*-butyl, *s*-butyl, *t*-butyl, vinyl or propenyl group, with the proviso that when each of R¹, R², and R³ are a hydrogen atom, R⁴ is not phenyl substituted. Even more preferably, R¹, R², R³ are each a hydrogen atom and R⁴ is an optionally substituted ethyl, *n*-butyl, *i*-butyl, *s*-butyl, *t*-butyl, or vinyl group, with the proviso that when each of R¹, R², and R³ are a hydrogen atom, R⁴ is not phenyl substituted.

Particularly preferred compounds of formula (I) obtained according to the method of the invention are those, wherein R¹, R², and R³, are each a hydrogen atom and R⁴ is an optionally substituted linear or branched C₁-C₆ alkyl or C₂-C₆ alkenyl group, such as methyl, ethyl, *n*-propyl, *i*-propyl, *n*-butyl, *i*-butyl, *s*-butyl, *t*-butyl, *n*-pentyl, *i*-pentyl, *t-*amyl, 1-ethyl-propyl, 1,2-dimethyl-propyl, 2,2-dimethyl-propyl, *n*-hexyl, 1-methyl-pentyl group, 2-methyl-pentyl group, 3-methyl-pentyl group, 4-methyl-pentyl group, 1,1-dimethyl-butyl, 1,2-dimethyl-butyl, 1,3-dimethyl-butyl, 2,2-dimethyl-butyl, 3,3-dimethyl-butyl, 1-ethyl-butyl, 2-ethyl-butyl, 1,1,2-trimethyl-propyl, 1,2,2-trimethyl-propyl, 1-ethyl-1-methyl-propyl or 1-ethyl-2-methyl-propyl, or an optionally substituted vinyl, 1-propenyl, 1-i-propenyl, 2-propenyl, 2-methyl-l-propenyl, 1-butenyl, 2-butenyl, 3-butenyl, 1-pentenyl, 2-pentenyl, 1-hexenyl, 2-hexenyl, 3-hexenyl, 2,4-pentadienyl group. Preferably, the optional substituent is not a phenyl group. More preferably, R¹, R², and R³, are each a hydrogen atom and R⁴ is an optionally substituted methyl, ethyl, *n*-propyl, *i*-propyl, *n-*butyl, *i*-butyl, *s*-butyl, *t*-butyl, vinyl or propenyl group, with the proviso that R⁴ is not phenyl substituted. Even more preferably, R¹, R², R³ are each a hydrogen atom and R⁴ is an optionally substituted ethyl, *n*-butyl, *i*-butyl, *s*-butyl, *t*-butyl, or vinyl group, with the proviso that R⁴ is not phenyl substituted.

The reaction of the compound of formula (III) with phosphoric acid is advantageously carried out at a temperature of room temperature to refluxing solvent, preferably 50°C to 80°C and even more preferably 70°C to 80°C

The organic solvent may be selected from the group comprising toluene, xylene, trimethylbenzene, cyclohexane, and methylcyclohexane. According to a preferred embodiment, the organic solvent is toluene.

The ratio of compound (I) to compound (II) depends among others on the reaction temperature, e.g. using toluene as organic solvent the ratio is of 20:80 at a temperature of 80°C and 50:50 at 110°C (reflux).

The phosphoric acid is advantageously used in an amount of 1 to 10% by weight of compound (III), preferably 2 to 5% by weight of compound (III). In a particularly preferred embodiment the phosphoric acid is concentrated phosphoric acid.

The compound of formula (II) may be separated from the compound of formula (I) by any suitable technique known in the art, such as distillation, HPLC, or column chromatography on silica gel.

The main advantage of the method of the invention is that the secondary product, i.e. the compound of formula (II), may easily be transformed into the corresponding compound of formula (I). The transformation of compound (II) into compound (II) is carried out by reacting compound (II) with trifluoromethanesulphonic acid in an organic solvent.

According to an advantageous variant of the method of the invention, the method of the invention therefore further comprises reacting the compound of formula (II) with trifluoromethanesulphonic acid (triflic acid). It is thus possible to selectively synthesize compounds of formula (I).

The reaction with trifluoromethanesulphonic acid is advantageously carried out at a temperature of about room temperature to refluxing solvent, preferably at a temperature of 50 to 80°C, even more preferably of 70 to 80°C.

According to an advantageous variant of the method of the invention, the transformation may either be carried out as a one-pot reaction, i.e. the trifluoromethanesulphonic acid is directly added to the reaction mixture obtained from reacting a compound of formula (III) with phosphoric acid as described above, or after separating the compound of formula (II) from the compound of formula (I) (two-pot reaction).

In the case where the transformation of compound (I) into compound (II) is carried out as a one-pot reaction, the conditions in respect of temperature and solvent are those set out above.

The amount of trifluoromethanesulphonic acid used is advantageously of 0.5 to 5% by weight of compound (III), preferably 0.7 to 3% by weight of compound (III), and even more preferably of 1 to 2% by weight of compound (III).

In the case where the compound of formula (II) is separated from the reaction mixture prior to its transformation into the corresponding compound of formula (I) (two-pot reaction), the separation may be carried out by any suitable technique known in the art, such as distillation, HPLC, or column chromatography on silica gel.

The organic solvent used in the two-pot reaction is selected from the group comprising toluene, xylene, trimethylbenzene, cyclohexane, and methylcyclohexane. According to a preferred embodiment, the organic solvent is toluene.

In the case of a two-pot reaction, the transformation is advantageously carried out at a temperature of about room temperature to refluxing solvent, preferably at a temperature of 50 to 80°C, even more preferably of 70 to 80°C.

In the two-pot reaction, the trifluoromethanesulphonic acid is advantageously used in an amount of 0.5 to 5% by weight of compound (II), preferably 0.7 to 3% by weight of compound (II), and even more preferably of 1 to 2% by weight of compound (II).

The starting compound of the method of the invention, i.e. the compound of formula (III) may easily be prepared by condensation of an appropriately substituted organometallic reagent (e.g. a Grignard reagent) with campholenaldehyde derived compounds (prepared according to known procedures from the person of the art).

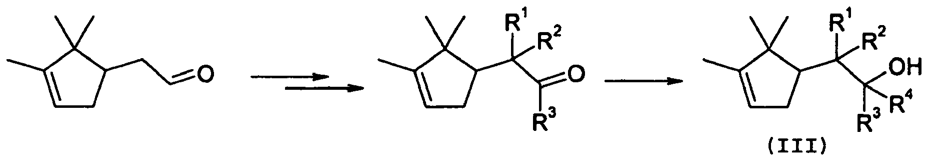

The invention is also directed to compounds of formula (II), wherein R1, R2, R3, and R4 are, independently, a hydrogen atom or an optionally substituted linear or branched C1-C6 alkyl or C2-C6 alkenyl group, provided that when each of R1, R2, and R3 are a hydrogen atom, R4 is not a phenyl substituted linear or branched C1-C6 alkyl or C2-C6 alkenyl group; and with the proviso that the compound of formula (II) is neither 2-(2,3,3-trimethyl-cyclopent-1-enyl)-ethanol, 1-(2,3,3-trimethyl-cyclopent-1-enyl)-butan-2-ol, nor 1-(2,3,3-trimethyl-cyclopent-1-enyl)-pent-3-en-2-ol.

A further object of the invention is a fragrant composition containing at least one compound of formula (I) or of formula (II) 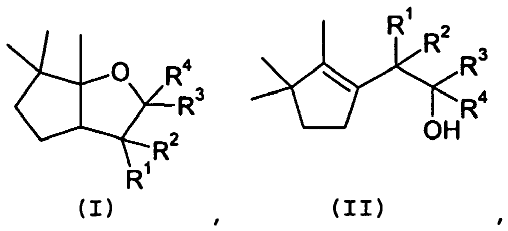 wherein R¹, R², R³, and R⁴ are, independently, a hydrogen atom or an optionally substituted linear or branched C₁-C₆ alkyl or C₂-C₆ alkenyl group, provided that when each of R¹, R², and R³ are a hydrogen atom, R⁴ is not a phenyl substituted linear or branched C₁-C₆ alkyl or C₂-C₆ alkenyl group; with the proviso that the compound of formula (I) is neither 6,6,6a-trimethyl-hexahydro-cyclopenta[b]furan, 6,6,6a-trimethyl-2-ethyl-hexahydro-cyclopenta[b]furan, nor 6,6,6a-trimethyl-2-propyl-hexahydro-cyclopenta[b]furan and that the compound of formula (II) is neither 2-(2,3,3-trimethyl-cyclopent-1-enyl)-ethanol, 1-(2,3,3-trimethyl-cyclopent-1-enyl)-butan-2-ol, nor 1-(2,3,3-trimethyl-cyclopent-1-enyl)-pent-3-en-2-ol.

This invention includes any composition comprising one or more isomers of a compound of formula (I) and/or of a compound of formula (II). According to a preferred embodiment, this invention relates to a composition comprising at least two or three isomers of a compound of formula (I) and/or of a compound of formula (II).

The fragrant composition of the invention may further comprise other perfuming ingredients, solvents, additives or fixatives, commonly used and that the man skilled in the art is able to choose in regard of the desired effect and the nature of the product to perfume.

A further object of the invention is the use of a compound of formula (I) or of formula (II), wherein R¹, R², R³, and R⁴ are, independently, a hydrogen atom or an optionally substituted linear or branched C₁-C₆ alkyl or C₂-C₆ alkenyl group, or of a fragrant composition as described above, in the perfumery field, for the preparation of perfumed bases and concentrates, fragrances, perfumes; topic compositions; cosmetic compositions, such as face and body creams, cleansers, facial treatments, talc powders, hair oils, shampoos, hair lotions, bath oils and salts, shower and bath gels, soaps, body anti-perspirants and deodorizers, pre-shave, shaving and post-shave creams and lotions, creams, toothpastes, mouth baths, pomades; and cleaning products, such as softeners, detergents, air deodorizers and household cleaning supplies.

The invention is also directed to the use of a compound of formula (I) or of formula (II), wherein R¹, R², R³, and R⁴ are, independently, a hydrogen atom or an optionally substituted linear or branched C₁-C₆ alkyl or C₂-C₆ alkenyl group, or of a fragrant composition as described above, as masking agent of odours, e.g. in pharmaceutical or cosmetic compositions.

The invention also provides the use of a compound of formula (I) or of formula (II), wherein R¹, R², R³, and R⁴ are, independently, a hydrogen atom or an optionally substituted linear or branched C₁-C₆ alkyl or C₂-C₆ alkenyl group, or of a fragrant composition as described above, in combination with other perfumed ingredients, solvents or additives or fixatives.

The compounds of the invention may be used in a concentration comprised in a range from 0.001% to 99% in weight, preferably from 0.1% to 50% in weight, more preferably from 0.1% to 30% in weight. It is known by the man skilled in the art that these values depend on the nature of the composition/article to be perfumed, the desired intensity of the perfume, and of the nature of the other ingredients present in said composition or article. According to a preferred embodiment of the invention, compounds are used in an olfactory effective amount.

### [DEFINITIONS]

The terms "fragrance" and "fragrant", as used herein, are used interchangeably whenever a compound or a mixture of compounds is referred to, which is intended to pleasantly stimulate the sense of smell.

The term "olfactory effective amount", as used herein, means a level or amount of fragrant compound present in a material at which the incorporated compound exhibits a sensory effect.

By the term "masking" is meant reducing or eliminating malodour perception generated by one or more molecules entering in the composition of a product.

The term "alkyl" or "alkyl group", in the present invention, means any linear or branched saturated hydrocarbon chain, having preferably 1, 2, 3, 4, 5 or 6 carbon atoms and herein referred to as C₁-C₆ alkyl group, such as for example methyl, ethyl, propyl, isopropyl, butyl, t-butyl, pentyl, or hexyl.

The term "alkenyl" or "alkenyl group", in the present invention, means any linear or branched mono or poly unsaturated hydrocarbon chain, having preferably 2, 3, 4, 5 or 6 carbon atoms and herein referred to as C₂-C₆ alkenyl group, such as for example ethenyl (vinyl), propenyl, butenyl, pentenyl, or hexenyl.

The term "isomer", in the present invention, means molecules having the same chemical formula, which means same number and types of atoms, but in which the atoms are arranged differently. The term "isomer" includes geometric isomers, optical isomers and stereoisomers.

The term "room temperature", as used herein, means a temperature of 18°C to 25°C.

### Example 1: Preparation of 2-isobutyl-6,6,6a-trimethyl-hexahydro-cyclopenta[b]furan and of 4-methyl-1-(2,3,3-trimethyl-cyclopent-1-enyl)-pentan-2-ol

A solution of 4-ethyl-1-(2,2,3-trimethyl-cyclopent-3-enyl)-pentan-2-ol (obtained from campholenaldehyde and 2-methyl-propylmagnesium bromide) in toluene with 3% weight of concentrated phosphoric acid is heated under reflux until completion of the reaction. After cooling down, the reaction mixture is washed with a saturated aqueous sodium bicarbonate solution and with brine. The organic layer is then dried over magnesium sulphate, filtered and the solvents are evaporated.

The single compounds are obtained by distillation of a (50:50) mixture of 2-isobutyl-6,6,6a-trimethyl-hexahydro-cyclopenta-[b]furan and of 4-methyl-1-(2,3,3-trimethyl-cyclopent-1-enyl)-pentan-2-ol.

### 2-Isobutyl-6,6,6a-trimethyl-hexahydro-cyclopenta[b]furan

Obtained as a (87:13) ratio of isomers.
Minty, blackcurrant (blackcurrant buds aspects), earthy odour with sweat aspects, reminiscent of bucchu essential oil.
Bp = 35°C/0.4 torr - 94°C/6 torr

### Main isomer:

**¹H-NMR** (200MHz, CDCl₃): δ 0.81 (t, 3H), 0.88 (d, 3H, *J =* 6.4Hz), 0.89 (d, 3H, *J* = 6.4Hz), 0.99 (s, 3H), 1.07 (s, 3H), 1.05-1.4 (m, 3H), 1.4-1.78 (m, 5H), 1.78-2.1 (m, 1H), 2.25-2.44 (m, 1H), 3.89 (dt, 1H, *J=* 6.3Hz, *J* = 12.0Hz).
**¹³ C-NMR** (50MHz, CDCl₃): δ 20.81, 22.17, 22.83, 23.10, 25.37, 25.83, 29.44, 40.41, 40.97, 46.39, 46.46, 47.69, 78.49, 94.26.

### Minor isomer:

**¹H-NMR** (200MHz, CDCl₃, selected data): δ 0.98 (s, 3H), 1.05 (s, 3H).
**¹³C-NMR** (50MHz, CDCl₃): δ 18.15, 22.16, 22.93, 23.23, 24.48, 25.73, 31.25, 38.43, 42.07, 44.72, 45.20, 47.94, 75.98, 94.06.
**IR** (film, cm⁻¹): 896w, 1010m, 1073m, 1092m, 1118m, 1368m, 1383m, 1444m, 1465m, 2870s, 2954s.

### 4-Methyl-1-(2,3,3-trimethyl-cyclopent-1-enyl)-pentan-2-ol

Light woody odour.
Bp = 65°C/0.4 torr
**¹H-NMR** (200MHz, CDCl₃): δ 0.89 (d, 1H, *J* = 3.0Hz), 0.93 (d, 1H, *J* = 3.1 Hz), 0.97 (s, 3H), 0.99 (s, 3H), 1.20 (ddd, 1H, *J =* 4.1Hz, *J =* 8.8Hz, *J* = 13.7Hz), 1.41 (ddd, 1H, *J =* 5.3Hz, *J* = 8.8Hz, *J* = 16.9Hz), 1.53 (t, 3H, *J =* 1.9Hz), 1.57-1.68 (m, 2H), 1.68-1.95 (m, 1H), 2.0-2.4 (m, 4H), 3.76 (qd, 1H, *J=* 4.4Hz, *J* = 12.9Hz).
**¹³C-NMR** (50MHz, CDCl₃): δ 9.62, 22.11, 23.49, 24.77, 26.16, 26.78, 32.92, 37.86, 38.86, 46.29, 47.0, 67.82, 129.93, 143.21.
**IR** (film, cm⁻¹): 1019w, 1074m, 1360m, 1383w, 1464m, 2843m, 2867m, 2932s, 2955s, 3360m br.
**MS** (EI, intensity (%)): 210(M+, 3), 195(1), 177(4), 109(100), 69(12), 43(12), 41(12).

### Example 2: Preparation of 2-isobutyl-6,6,6a-trimethyl-hexahydro-cyclopenta[b]furan from 4-methyl-1-(2,3,3-trimethyl-cyclopent-1-enyl)-pentan-2-ol

A solution of 4-methyl-1-(2,3,3-trimethyl-cyclopent-1-enyl)-pentan-2-ol in toluene is heated at 80°C and 1% weight of triflic acid is added. After completion of the reaction, the reaction mixture is cooled down and washed with a saturated aqueous sodium bicarbonate solution and with brine. The organic layer is then dried over magnesium sulphate, filtered and the solvents are evaporated. The crude 2-isobutyl-6,6,6a-trimethyl-hexahydro-cyclopenta[b]furan is purified by distillation.

### Example 3: "One pot" preparation of 2-isobutyl-6,6,6a-trimethyl-hexahydro-cyclopenta[b]furan

A solution of 4-methyl-1-(2,2,3-trimethyl-cyclopent-3-enyl)-pentan-2-ol (obtained from campholenaldehyde and 2-methyl-propylmagnesium bromide) in toluene with 3% weight of phosphoric acid is heated at 80°C until 4-methyl-1-(2,2,3-trimethyl-cyclopent-3-enyl)-pentan-2-ol is totally converted into 4-methyl-1-(2,3,3-trimethyl-cyclopent-1-enyl)-pentan-2-ol. After cooling down to room temperature, 1% weight of triflic acid is added and the reaction mixture is further stirred until 4-methyl-1-(2,3,3-trimethyl-cyclopent-1-enyl)-pentan-2-ol is converted into 2-isobutyl-6,6,6a-trimethyl-hexahydro-cyclopenta[b]furan.

After completion of the reaction, the work-up is carried out according to example 2.

### Example 4: Preparation of 1-(2,3,3-trimethyl-cyclopent-1-enyl)-but-3-en-2-ol

A solution of 1-(2,2,3-trimethyl-cyclopent-3-enyl)-but-3-en-2-ol (obtained from campholenaldehyde and vinylmagnesium bromide) in toluene with 3% weight of concentrated phosphoric acid is heated at 80°C until completion of the reaction. After cooling down, the reaction mixture is washed with a saturated aqueous sodium bicarbonate solution and with brine. The organic layer is then dried over magnesium sulphate, filtered and the solvents are evaporated.

The crude product consists in a (20:80) mixture of 6,6,6a-trimethyl-2-vinyl-hexahydro-cyclopenta[b]furan and 1-(2,3,3-trimethyl-cyclopent-1-enyl)-but-3-en-2-ol.

### 1-(2,3,3-Trimethyl-cyclopent-1-enyl)-but-3-en-2-ol

**¹H-NMR** (200MHz, CDCl₃): δ 0.97 (s, 3H), 0.99 (s, 3H), 1.0-1.4 (m, 1H), 1.4-1.6 (m, 3H), 1.6-2 (m, 2H), 2.0-2.4 (m, 3H), 4.2 (m, 1H), 5.09 (td, 1H, *J =* 1.5Hz, *J* = 10.4Hz), 5.24 (td, 1H, *J =* 1.5Hz, *J =* 17.2Hz), 5.87 (ddd, 1H, *J =* 5.8Hz, *J = 10.4Hz, J=* 17.2Hz). **¹³C-NMR** (50MHz, CDCl₃): δ 9.63, 26.22, 26.65, 32.96, 37.15, 38.84, 46.95, 71.09, 114.26, 137.81, 140.66, 143.36.
**MS** (EI, intensity (%)): 180(M+, 4), 165(12), 147(11), 124(17), 123(68), 109(92), 95(18), 93(20), 91(24), 81(100), 79(23), 77(21), 67(38), 57(36), 55(33), 43(15), 41(28), 39(15).

### Example 5: Preparation of 6,6,6a-trimethyl-2-vinyl-hexahydro-cyclopenta[b]furan

1-(2,3,3-Trimethyl-cyclopent-1-enyl)-but-3-en-2-ol, obtained in example 4, is converted into 6,6,6a-trimethyl-2-vinyl-hexahydro-cyclopenta[b]furan according to example 2.

### 6,6,6a-Trimethyl-2-vinyl-hexahydro-cyclopenta[b]furan

Obtained in a (80:20) ratio of isomers.
Woody, camphoraceous.
Bp = 65°C/5 torr

### Major isomer:

**¹H-NMR** (200MHz, CDCl₃): δ 0.83 (s, 3H), 1.02 (s, 3H), 1.08-1.47 (m, 2H), 1.12 (s, 3H), 1.47-1.75 (m, 1H), 1.75-2.5 (m, 4H), 4.28 (dd, 1H, *J =* 7.0Hz, *J* = 16.6Hz), 5.05 (ddd, 1H, *J =* 0.8Hz, *J =* 1.8Hz, *J =* 10.1Hz), 5.21 (ddd, 1H, *J =* 0.9Hz, *J* = 1.8Hz, *J =* 17.1Hz), 5.79 (ddd, 1H, *J =* 7.2Hz, *J =* 10.1Hz, *J* = 17.2Hz).
**¹³C-NMR** (50MHz, CDCl₃): δ 20.36, 22.12, 25.38, 29.09, 40.13, 41.08, 46.39, 47.84, 81.44, 95.61, 115.32, 140.55.

### Minor isomer (selected data):

**¹H-NMR** (200MHz, CDCl₃): δ 0.89 (s, 3H), 1.03 (s, 3H), 1.1 (s, 3H), 2.5-2.7 (m, 1H), 4.3 (m, 1H), 5.04 (ddd, 1H*, J* = 1.1Hz*, J* = 1. 8Hz, *J* = 10.3Hz), 5.23 (ddd, 1H*, J* = 1.3Hz*, J =* 1.8Hz, *J* = 17.2Hz), 5.87 (ddd, 1H, *J* = 6.6Hz, *J* = 10.4Hz, *J* = 17Hz).
**¹³C-NMR** (50MHz, CDCl₃): δ 18.25, 22.23, 24.63, 30.84, 38.4, 42.11, 44.86, 48.15, 78.71, 95.16, 114.44, 139.42.

### Example 6: "One pot" preparation of 6,6,6a-trimethyl-2-vinyl-hexahydro-cyclopenta[b]furan

1-(2,2,3-Trimethyl-cyclopent-3-enyl)-but-3-en-2-ol (obtained from campholenaldehyde and vinylmagnesium bromide) was prepared according to example 3, using 5% weight of phosphoric acid and 2% weight of triflic acid.

### Example 7: Preparation of 2-ethyl-6,6,6a-trimethyl-hexahydro-cyclopenta[b]furan and of 1-(2,3,3-Trimethyl-cyclopent-1-enyl)-butan-2-ol

The single compounds were obtained by distillation of a (50:50) mixture prepared, *via* ethylmagnesium bromide with campholenaldehyde, according to example 1.

### 2-Ethyl-6,6,6a-trimethyl-hexahydro-cyclopenta[b]furan

2 isomers in a (85:15) ratio, having an intense camphoraceous, green, animalic and cassis-like odour, reminiscent of bucchu essential oil.
Bp = 65°C/5 torr

### Major isomer:

**¹H-NMR** (500MHz, CDCl₃): δ 0.82 (s, 3H), 0.87 (t, 3H, *J* = 7.5Hz), 1.0 (s, 3H), 1.09 (s, 3H), 1.17-1.25 (m, 1H), 1.32 (dd, 1H*, J* = 8.2Hz, *J* = 12.1Hz), 1.40 (td, 1H, *J* = 7.4Hz*, J* = 13.2Hz), 1.55-1.78 (m, 4H), 1.97 (m, 1H), 2.37 (m, 1H), 3.79 (td, 1H, J=5.3Hz, J=12.4Hz).
**¹³C-NMR** (50MHz, CDCl₃): δ 10.09, 20.61, 22.15, 25.39, 29.41, 29.46, 39.69, 40.35, 46.4, 47.68, 81.46, 94.56.

### Minor isomer:

**¹H-NMR** (500MHz, CDCl₃, selected data): δ 0.89 (t, 1H, *J* = 7.6Hz), 2.29-2.33 (m, 1H).
**¹³C-NMR** (50MHz, CDCl₃): δ 10.09, 18.17, 22.15, 24.52, 28.50, 31.21, 38.50, 40.80, 44.82, 47.84, 78.79, 94.38.
**IR** (film, cm⁻¹): 985m, 1079m, 1117m, 1368m, 1383m, 1453m, 1465m, 2870s, 2959s. **MS** (EI, intensity (%)): 182(21), 167(2), 153(2), 126(9), 125(100), 112(10), 111(6), 109(8), 83(9), 81(5), 71(8), 69(8), 55(18), 43(37), 41(20).

### 1-(2,3,3-Trimethyl-cyclopent-1-enyl)-butan-2-ol

Woody, camphoraceous odour, with mouldy aspects.
Bp = 91-92°C/5 torr
**¹H-NMR** (200MHz, CDCl₃): δ 0.93-1.0 (m, 3H), 1.35-1.51 (m, 2H), 1.53 (s, 3H), 1.55-1.68 (m, 3H), 2.10-2.23 (m, 2H), 2.23-2.32 (m, 1H), 3.57-3.65 (m, 1H).
**¹³C-NMR** (50MHz, CDCl₃): δ 9.59, 10.06, 26.18, 26.74, 29.72, 32.90, 36.88, 38.85, 46.97, 71.15, 129.95, 143.07.
**IR** (film, cm⁻¹):971m, 1114m, 1359w, 1460m, 2865m, 3031s, 3058s, 3383m br.
**MS** (EI, intensity (%)): 182(M+, 8), 167(9), 149(8), 109(100), 59(11).

### Example 8: Preparation of 2-ethyl-6,6,6a-trimethyl-hexahydro-cyclopenta[b]furan from 1-(2,3,3-trimethyl-cyclopent-1-enyl)-butan-2-ol

1-(2,3,3-Trimethyl-cyclopent-1-enyl)-butan-2-ol was converted into 2-ethyl-6,6,6a-trimethyl-hexahydro-cyclopenta[b]furan according to example 2.

### Example 9: Preparation of 2-sec-butyl-6,6,6a-trimethyl-hexahydro-cyclopenta[b]furan

2-sec-Butyl-6,6,6a-trimethyl-hexahydro-cyclopenta[b]furan was prepared from 3-methyl-1-(2,2,3-trimethyl-cyclopent-3-enyl)-pentan-2-ol (obtained from campholenaldehyde and 1-methyl-propylmagnesiumbromide) according to example 3, using 3% weight of phosphoric acid at 100°C in toluene, then 1% weight of triflic acid at 80°C.

It was obtained as a 45:45:5:5 ratio of isomers.
Blackcurrant, blackcurrant buds effect, camphoraceous, citral.
Bp = 96-98°C/ 8 torr

### Major isomers:

**¹H-NMR** (200MHz, CDCl₃): δ 0.76-0.9 (m, 3H), 0.82 (s, 3H), 0.90 (d, 1H, *J* = 6.7Hz), 0.99 (s, 3H), 1.05 & 1.06 (s, 3H), 1.1-1.35 (m, 2H), 1.35-1.65 (m, 4H), 1.65-1.85 (m, 2H), 1.85-2.1 (m, 1H), 2.27-2.41 (m, 1H), 3.66 (td, 1H, *J*= 5.5Hz, *J*= 11.1Hz).
**¹³C-NMR** (50MHz, CDCl₃): δ 11.55 & 11.64, 14.13 & 15.62, 20.32, 22.16, 25.06 & 26.73, 25.41, 29.49, 36.68 & 37.09, 39.79, 40.49, 47.43 & 47.48, 84.12 & 84.42, 93.91 & 94.07.

### Minor isomers:

**¹H-NMR** (200MHz, CDCl₃, selected data): δ 1.02 & 1.03 (s, 3H), 3.47 (m, 1H).
¹**³C-NMR** (50MHz, CDCl₃, selected data): δ 81.09 & 81.5.
**IR** (film, cm⁻¹): 896w, 1009m, 1077s, 1118m, 1370m, 1382m, 1458m, 1464m, 2873s, 2960s.

### Example 10: Preparation of 2-butyl-6,6,6a-trimethyl-hexahydro-cyclopenta[b]furan

2-Butyl-6,6,6a-trimethyl-hexahydro-cyclopenta[b]furan was prepared from 1-(2,2,3-Trimethyl-cyclopent-3-enyl)-hexan-2-ol 1-(2,3,3-Trimethyl-cyclopent-1-enyl)-hexan-2-ol (obtained from campholenaldehyde and butyllithium) according to example 3.

It was obtained as a 90:10 ratio of isomers.
Blackcurrant, blackcurrant buds, red berries.
Bp = 93-94°C / 7 torr

### Major isomer:

**¹H-NMR** (200MHz, CDCl₃): δ 0.81 (s, 3H), 0.82-0.94 (m, 3H), 1.0 (s, 3H), 1.08 (s, 3H), 1.10-1.45 (m, 6H), 1.5-1.8 (m, 4H), 1.8-2.1 (m, 2H), 2.3-2.45 (m, 1H), 3.82 (m, *J* = 5.9Hz, *J* = 12.2Hz).
**¹³C-NMR** (50MHz, CDCl₃): δ 14.05, 20.68, 22.146, 22.88, 25.376, 28.32, 29.40, 36.72, 40.35 (2C), 46.38, 47.71, 80.27, 94.42.

### Minor isomer:

**¹H-NMR** (200MHz, CDCl₃, selected data): δ 3.55-3.75 (m, 1H).
**¹³C-NMR** (50MHz, CDCl₃, selected data): δ 46.98, 47.90, 77.61, 94.23.
**IR** (film, cm⁻¹): 899w, 1010m, 1074m, 1090m, 1117m, 1368m, 1382m, 1458m, 1464m, 2868s, 2936s, 2956s.

### Example 11: Fragrance composition containing 2-ethyl-6,6,6a-trimethyl-hexahydro-cyclopenta[b]furan

A blackcurrant type accord was prepared as described from the following ingredients:

| **Ingredient** | **Accord 1** | **Accord 2** | **Accord 3** | **Accord 4** |
|---|---|---|---|---|
| DIMETHYLOCTANOL | 15 | 15 | 15 | 15 |
| DYNASCONE® 10 | 20 | 20 | 20 | 20 |
| JASMOLACTONE | 10 | 10 | 10 | 10 |
| METHYL DIHYDRO JASMONATE | 120 | 120 | 120 | 120 |
| NEROL | 20 | 20 | 20 | 20 |
| ROSE OXIDE 10% DPG | 40 | 40 | 40 | 40 |
| STRAWBERRY FURANONE 30% Alc. | 15 | 15 | 15 | 15 |
| TRIPLAL® | 15 | 15 | 15 | 15 |
| VELOUTONE® | 20 | 20 | 20 | 20 |
| DIPROPYLENE GLYCOL | 725 | 715 | 675 | 625 |
| 2-ethyl-6,6,6a-trimethyl-hexahydro-cyclopenta[b]furan | - | 10 | 50 | 100 |

These 4 compositions, containing different amount of the bicyclic ether, were used in a shower gel at usual dilution, known from the person of the art, and the samples containing the compound of formula (I) showed a greener metallic note, with earthy aspects.

### Example 12: Fragrance composition containing 2-ethyl-6,6,6a-trimethyl-hexahxdro-cyclopenta[b]furan or 2-isobutyl-6,6,6a-trimethyl-hexahydro-cyclopenta[b]furan

To a blackcurrant type accord, prepared as described from the following ingredients (column 2), is added 2-ethyl-6,6,6a-trimethyl-hexahydro-cyclopenta[b]furan (column 3) or 2-isobutyl-6,6,6a-trimethyl-hexahydro-cyclopenta[b]furan (column 4). The 2 new compositions are compared to the primary one to evaluate the impact of the added ingredient.

| **Ingredient** | **Accord 1** | **Accord 2** | **Accord 3** | **Accord 4** | **Accord 5** |
|---|---|---|---|---|---|
| TEREBENTHINE ESS | 15 | 15 | 15 | 15 | 15 |
| PINENE BETA | 100 | 100 | 100 | 100 | 100 |
| ORANGE TERPENES BRESIL | 30 | 30 | 30 | 30 | 30 |
| HEXYLE ACETATE | 3 | 3 | 3 | 3 | 3 |
| LINALOOL | 100 | 100 | 100 | 100 | 100 |
| VERDOX | 20 | 20 | 20 | 20 | 20 |
| VERTENEX | 30 | 30 | 30 | 30 | 30 |
| TERPINEOL | 12 | 12 | 12 | 12 | 12 |
| TERPENYL ACETATE | 25 | 25 | 25 | 25 | 25 |
| CIS-3-HEXENOL | 1 | 1 | 1 | 1 | 1 |
| TRIPLAL | 10 | 10 | 10 | 10 | 10 |
| CITRONELLOL | 30 | 30 | 30 | 30 | 30 |
| HEXYLCINNAMIQUE ALDEHYDE | 50 | 50 | 50 | 50 | 50 |
| METHYL DIHYDROJASMONATE | 150 | 150 | 150 | 150 | 150 |
| VANILLINE | 1 | 1 | 1 | 1 | 1 |
| PHENYLETHYL ALCOHOL | 10 | 10 | 10 | 10 | 10 |
| GERANIOL | 30 | 30 | 30 | 30 | 30 |
| ALDEHYDE C14 | 2 | 2 | 2 | 2 | 2 |
| *GAMMA*-DECALACTONE | 1 | 1 | 1 | 1 | 1 |
| BENZYL ACETATE | 10 | 10 | 10 | 10 | 10 |
| IONONE BETA | 10 | 10 | 10 | 10 | 10 |
| FRAMBINONE | 1 | 1 | 1 | 1 | 1 |
| FRUCTONE | 4 | 4 | 4 | 4 | 4 |
| EUCALYPTOL | 1 | 1 | 1 | 1 | 1 |
| DIHYDROFLORIFFONE | 1 | 1 | 1 | 1 | 1 |
| ETHYL MALTOL 5% ALCOOL BENZYLIQUE | 15 | 15 | 15 | 15 | 15 |
| DULCINYL | 5 | 5 | 5 | 5 | 5 |
| DPG | 333 | 313 | 303 | 303 | 303 |
| 2-Ethyl-6,6,6a-trimethyl-hexahydro-cyclopenta[b]furan | - | 20 | - | - | - |
| 2-sec-Butyl-6,6,6a-trimethyl-hexahydro-cyclopenta[b]furan | - | - | 30 | - | - |
| 2-Butyl-6,6,6a-trimethyl-hexahydro-cyclopenta[b]furan | - | - | - | 30 | - |
| 2-Isobutyl-6,6,6a-trimethyl-hexahydro-cyclopenta[b]furan | - | - | - | - | 30 |

Adding 2-ethyl-6,6,6a-trimethyl-hexahydro-cyclopenta[b]furan to the composition adds lift to the odour as well as power. In addition it gives a fresh and sparkling Bucchu effect, and the berry aspect is enhanced.

Adding 2-sec-butyl-6,6,6a-trimethyl-hexahydro-cyclopenta[b]-furan to the composition gives power to the odour and increases the red fruits aspect, it also gives a lift to the lactonic and vanilla notes imparting a "gourmand" aspect.

Adding 2-butyl-6,6,6a-trimethyl-hexahydro-cyclopenta[b]-furan to the composition really increases the red berries aspect, nevertheless with a slight decrease in the odour power. However, the accord 4 has thus a longer lasting note.

Adding 2-isobutyl-6,6,6a-trimethyl-hexahydro-cyclopenta[b]-furan to the composition gives power to the odour and this accord has now a very powerful blackcurrant buds aspect.

## Claims

1. A compound of general formula (I): 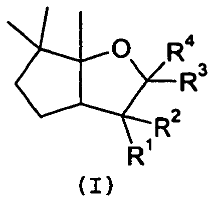 wherein
R¹, R², R³, and R⁴ are, independently, a hydrogen atom or an optionally substituted linear or branched C₁-C₆ alkyl or C₂-C₆ alkenyl group,
provided that when each of R¹, R², and R³ are a hydrogen atom, R⁴ is not a phenyl substituted linear or branched C₁-C₆ alkyl or C₂-C₆ alkenyl group; and
with the proviso that the compound of formula (I) is neither 6,6,6a-trimethyl-hexahydro-cyclopenta[b]furan, 6,6,6a-trimethyl-2-ethyl-hexahydro-cyclopenta[b]furan, 6,6,6a-trimethyl-2-propyl-hexahydro-cyclopenta[b]furan, nor 3-methyl-3-(6,6,6a-trimethyl-hexahydro-cyclopenta[b]-furan-2-yl)-butanone.

2. The compound of claim 1, wherein R¹, R², R³, and R⁴ are, independently, a hydrogen atom or an optionally substituted methyl, ethyl, *n*-propyl, *i*-propyl, *n*-butyl, *i-*butyl, *s*-butyl, *t*-butyl, *n*-pentyl, *i*-pentyl, *t*-amyl, 1-ethyl-propyl, 1,2-dimethyl-propyl, 2,2-dimethyl-propyl, n-hexyl, 1-methyl-pentyl group, 2-methyl-pentyl group, 3-methyl-pentyl group, 4-methyl-pentyl group, 1,1-dimethyl-butyl, 1,2-dimethyl-butyl, 1,3-dimethyl-butyl, 2,2-dimethyl-butyl, 3,3-dimethyl-butyl, 1-ethyl-butyl, 2-ethyl-butyl, 1,1,2-trimethyl-propyl, 1,2,2-trimethyl-propyl, 1-ethyl-1-methyl-propyl or 1-ethyl-2-methyl-propyl, or an optionally substituted vinyl, 1-propenyl, 1-*i*-propenyl, 2-propenyl, 2-methyl-1-propenyl, 1-butenyl, 2-butenyl, 3-butenyl, 1-pentenyl, 2-pentenyl, 1-hexenyl, 2-hexenyl, 3-hexenyl, 2,4-pentadienyl group, with the proviso that the optional substituent is not a phenyl group.

3. The compound of claim 1 or 2, wherein R¹, R², and R³, are each a hydrogen atom and R⁴ is an optionally substituted linear or branched C₁-C₆ alkyl or C₂-C₆ alkenyl group, with the proviso that the optional substituent is not a phenyl group.

4. The compound of formula (I), selected from the group consisting of 2-isobutyl-6,6,6a-trimethyl-hexahydro-cyclopenta[b]furan, 2-sec-butyl-6,6,6a-trimethyl-hexahydro-cyclopenta[b]furan, 2-butyl-6,6,6a-trimethyl-hexahydro-cyclopenta[b]furan, 6,6,6a-trimethyl-2-vinyl-hexahydro-cyclopenta[b]furan.

5. A method of preparing a compound of general formula (I): 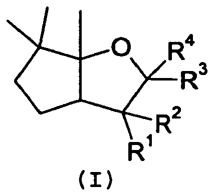 wherein
R¹, R², R³, and R⁴ are, independently, a hydrogen atom or an optionally substituted linear or branched C₁-C₆ alkyl or C₂-C₆ alkenyl group,
the method comprising reacting a compound of formula (III): 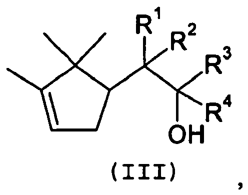 wherein
R¹, R², R³, and R⁴ are defined as in respect of formula (I),
with phosphoric acid in an organic solvent so as to obtain a mixture comprising a compound of formula (I) and a compound of formula (II): 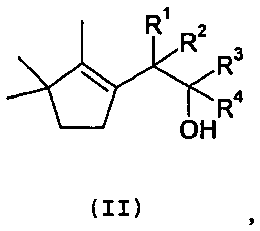 wherein
R¹, R², R³, and R⁴ are defined as in respect of formula (I).

6. The method of claim 5, wherein the organic solvent is selected from the group comprising toluene, xylene, trimethylbenzene, cyclohexane, and methylcyclohexane.

7. The method of claim 5 or 6, wherein the phosphoric acid is used in an amount of 1 to 10% by weight of compound (III).

8. The method of any one of claims 5 to 7, further comprising reacting the compound of formula (II) with trifluoromethanesulphonic acid.

9. The method of claim 8, comprising, prior to the reaction with trifluoromethanesulphonic acid, the separation and purification of the compound of formula (II).

10. The method of claim 9, wherein the trifluoromethanesulphonic acid is used in an amount of 0.5 to 2% by weight of compound (II).

11. A compound of general formula (II): 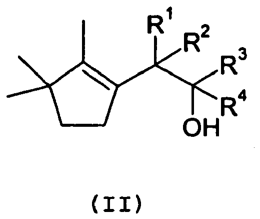 wherein
R¹, R², R³, and R⁴ are, independently, a hydrogen atom or an optionally substituted linear or branched C₁-C₆ alkyl or C₂-C₆ alkenyl group,
provided that when each of R¹, R², and R³ are a hydrogen atom, R⁴ is not a phenyl substituted linear or branched C₁-C₆ alkyl or C₂-C₆ alkenyl group; and
with the proviso that the compound of formula (II) is neither 2-(2,3,3-trimethyl-cyclopent-1-enyl)-ethanol, 1-(2,3,3-trimethyl-cyclopent-1-enyl)-butan-2-ol, nor 1-(2,3,3-trimethyl-cyclopent-1-enyl)-pent-3-en-2-ol.

12. Fragrant composition containing at least one compound according to any one of claims 1 to 4 and 11.

13. Use of a compound of formula (I) or of formula (II): 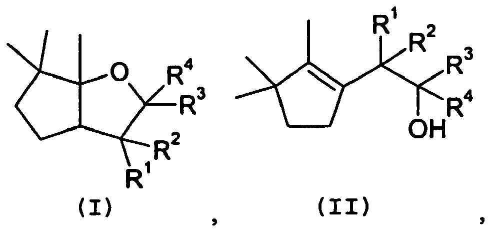 wherein R¹, R², R³, and R⁴ are, independently, a hydrogen atom or an optionally substituted linear or branched C₁-C₆ alkyl or C₂-C₆ alkenyl group, or of a composition according to Claim 12, in the field of perfumery, for the preparation of perfumed bases and concentrates, fragrances, perfumes; topic compositions; cosmetic compositions, such as face and body creams, cleansers, facial treatment, talc powders, hair oils, shampoos, hair lotions, bath oils and salts, shower and bath gels, soaps, body anti-perspirants and deodorizers, pre-shave, shaving and post-shave creams and lotions, creams, toothpastes, mouth baths, and pomades; and cleaning products, such as softeners, detergents, air deodorizers and household cleaning supplies.

14. Use of a compound of formula (I) or of formula (II): 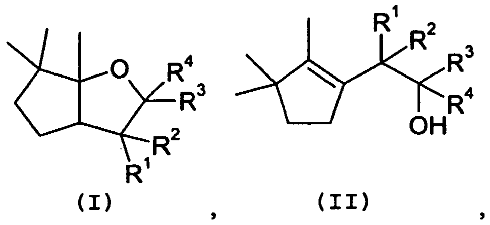 wherein R¹, R², R³, and R⁴ are, independently, a hydrogen atom or an optionally substituted linear or branched C₁-C₆ alkyl or C₂-C₆ alkenyl group, or of a composition according to claim 12, as masking agent of odours.

## Patentansprüche

1. Verbindung der allgemeinen Formel (I): 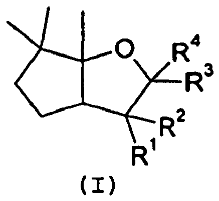 wobei
R¹, R², R³ und R⁴ unabhängig voneinander ein Wasserstoffatom oder eine optional substituierte lineare oder verzweigte C₁-C₆-Alkyl- oder C₂-C₆-Alkenylgruppe sind, unter der Maßgabe, dass, wenn jeder von R¹, R² und R³ ein Wasserstoffatom ist, R⁴ keine phenylsubstituierte lineare oder verzeigte C₁-C₆-Alkyl- oder C₂-C₆-Alkenylgruppe ist; und
unter der Maßgabe, dass die Verbindung der Formel (I) weder 6,6,6a-Trimethyl-hexahydro-cyclopenta[b]furan, 6,6,6a-Trimethyl-2-ethyl-hexahydro-cyclopenta[b]furan, 6,6,6a-Trimethyl-2-propyl-hexahydro-cyclopenta[b]furan noch 3-Methyl-3-(6,6,6a-Trimethyl-hexahydro-cyclopenta[b]-furan-2-yl)-butanon ist.

2. Verbindung nach Anspruch 1, wobei R¹, R², R³ und R⁴ unabhängig voneinander ein Wasserstoffatom oder ein optional substituiertes Methyl, Ethyl, *n*-Propyl, *i*-Propyl, *n*-Butyl, *i*-Butyl, *s*-Butyl, *t*-Butyl, *n*-Pentyl, *i*-Pentyl, *t*-Amyl, 1-Ethylpropyl, 1,2,-Dimethylpropyl, 2,2,-Dimethylpropyl, *n*-Hexyl, eine 1-Methylpentyl-Gruppe, eine 2-Methylpentyl-Gruppe, eine 3-Methylpentyl-Gruppe, eine 4-Methylpentyl-Gruppe, 1,1-Dimethylbutyl, 1,2-Dimethylbutyl, 1,3-Dimethylbutyl, 2,2-Dimethylbutyl, 3,3-Dimethylbutyl, 1-Ethylbutyl, 2-Ethylbutyl, 1,1,2-Trimethylpropyl, 1,2,2,-Trimethylpropyl, 1-Ethyl-1-methylpropyl oder 1-Ethyl-2-methylpropyl, oder ein optional substituiertes Vinyl, 1-Propenyl, 1-*i*-Propenyl, 2-Propenyl, 2-Methyl-1-propenyl, 1-Butenyl, 2-Butenyl, 3-Butenyl, 1-Pentenyl, 2-Pentenyl, 1-Hexenyl, 2-Hexenyl, 3-Hexenyl, eine 2,4-Pentadienylgruppe sind, unter der Maßgabe, dass der optionale Substituent keine Phenylgruppe ist.

3. Verbindung nach Anspruch 1 oder 2, wobei R¹, R² und R³ jeweils ein Wasserstoffatom sind und R⁴ ein optional substituierte lineare oder verzweigte C₁-C₆-Alkyl- oder C₂-C₆-Alkenylgruppe ist, unter der Maßgabe, dass der optionale Substituent keine Phenylgruppe ist.

4. Verbindung der Formel (I), ausgewählt aus der Gruppe bestehend aus 2-Isobutyl-6,6,6a-trimethyl-hexahydro-cyclopenta[b]furan, 2-*sec*-Butyl-6,6,6a-trimethyl-hexahydro-cyclopenta[b]furan, 2-Butyl-6,6,6a-trimethyl-hexahydro-cyclopenta[b]furan, 6,6,6a-Trimethyl-2-vinyl-hexahydro-cyclopenta[b]furan.

5. Verfahren zum Herstellen einer Verbindung der allgemeinen Formel (I): 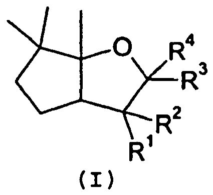 wobei
R¹, R², R³ und R⁴ unabhängig voneinander ein Wasserstoffatom oder eine optional substituierte lineare oder verzweigte C₁-C₆-Alkyl- oder C₂-C₆-Alkenylgruppe sind, wobei das Verfahren Umsetzen einer Verbindung der Formel (III): 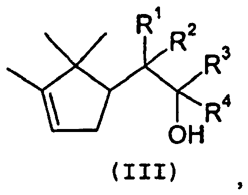 wobei R¹, R² und R³ und R⁴ wie in Formel (I) definiert sind,
mit Phosphorsäure in einem organischen Lösungsmittel umfasst, so dass eine Mischung erhalten wird, umfassend eine Verbindung der Formel (I) und eine Verbindung der Formel (II): 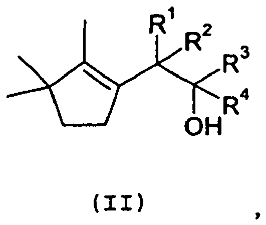 wobei
R¹, R² und R³ und R⁴ wie in Formel (I) definiert sind.

6. Verfahren nach Anspruch 5, wobei das organische Lösungsmittel ausgewählt ist aus der Gruppe umfassend Toluol, Xylol, Trimethylbenzol, Cyclohexan und Methylcyclohexan.

7. Verfahren nach Anspruch 5 oder 6, wobei die Phosphorsäure in einer Menge von 1 bis 10 Gew.-% bezogen auf Verbindung (III) verwendet wird.

8. Verfahren nach einem der Ansprüche 5 bis 7, weiterhin umfassend Umsetzen der Verbindung der Formel (II) mit Trifluormethansulfonsäure.

9. Verfahren nach Anspruch 8, umfassend vor der der Reaktion mit Trifluormethansulfansäure, die Abtrennung und Reinigung der Verbindung der Formel (II).

10. Verfahren nach Anspruch 9, wobei die Trifluormethansulfonsäure in einer Menge von 0,5 bis 2 Gew.-% bezogen auf Verbindung (II) verwendet wird.

11. Verbindung der allgemeinen Formel (II): 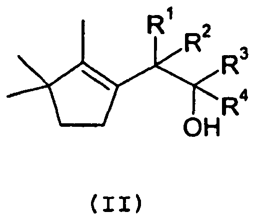 wobei
R¹, R², R³ und R⁴ unabhängig voneinander ein Wasserstoffatom oder eine optional substituierte lineare oder verzweigte C₁-C₆-Alkyl- oder C₂-C₆-Alkenylgruppe sind, unter der Maßgabe, dass, wenn jeder von R¹, R² und R³ ein Wasserstoffatom ist, R⁴ keine phenylsubstituierte lineare oder verzeigte C₁-C₆-Alkyl- oder C₂-C₆-Alkenylgruppe ist; und
unter der Maßgabe, dass die Verbindung der Formel (II) weder 2-(2,3,3-Trimethyl-cyclopent-1-enyl)-ethanol, 1-(2,3,3-Trimethyl-cyclopent-1-enyl)-butan-2-ol noch 1-(2,3,3-Trimethyl-cyclopent-1-enyl)-pent-3-en-2-ol ist.

12. Duftzusammensetzung, enthaltend wenigstens eine Verbindung nach einem der Ansprüche 1 bis 4 und 11.

13. Verwendung einer Verbindung der Formel (I) oder der Formel (II): 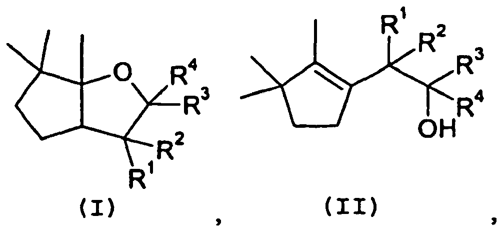 wobei R¹, R², R³ und R⁴ unabhängig voneinander ein Wasserstoffatom oder eine optional substituierte lineare oder verzweigte C₁-C₆-Alkyl- oder C₂-C₆-Alkenylgruppe sind, oder einer Zusammensetzung nach Anspruch 12 auf dem Gebiet des Parfümeriewesens zur Herstellung von parfümierten Basismitteln und Konzentraten, Düften, Parfümen; topischen Zusammensetzungen; kosmetischen Zusammensetzungen, z.B. Gesichts- und Körpercremes, Reiniger, Gesichtsbehandlungen, Talkpuder, Haaröle, Shampoos, Haarlotionen, Badöle und -salze, Dusch- und Badegele, Seifen, Körperantitraspirantien und -deodorants, Cremes und Lotionen für die Vorrasur, Rasur und Nachrasur, Cremes, Zahnpasten, Mundwässer und Pomaden; und von Reinigungsprodukten, z.B. Weichmacher, Detergenzien, Luftdesodorierer und Reinigungsmittel für den Haushalt.

14. Verwendung einer Zusammensetzung der Formel (I) oder der Formel (II): 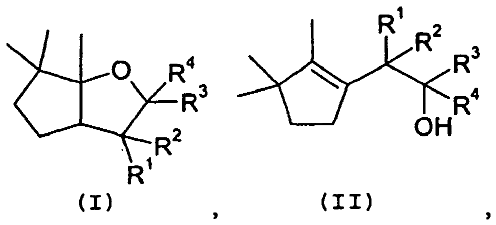 wobei R¹, R², R³ und R⁴ unabhängig voneinander ein Wasserstoffatom oder eine optional substituierte lineare oder verzweigte C₁-C₆-Alkyl- oder C₂-C₆-Alkenylgruppe sind, oder einer Zusammensetzung nach Anspruch 12 als Mittel zur Maskierung von Gerüchen.

## Revendications

1. Composé de formule générale (I) : 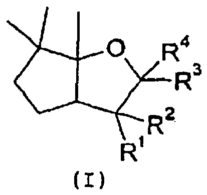 dans laquelle
R¹, R² R³ et R⁴ sont, indépendamment, un atome d'hydrogène ou un groupe alkyle en C₁ à C₆ ou alcényle en C₂ à C₆ linéaire ou ramifié facultativement substitué,
à condition que lorsque chacun de R¹ R² et R³ est un atome d'hydrogène, R⁴ ne soit pas un groupe alkyle en C₁ à C₆ ou alcényle en C₂ à C₆ linéaire ou ramifié substitué par phényle ; et
à condition que le composé de formule (I) ne soit ni le 6,6,6a-triméthyl-hexahydro-cyclopenta[b]furane, le 6,6,6a-triméthyl-2-éthyl-hexahydro-cyclopenta[b]furane, le 6,6,6a-triméthyl-2-propyl-hexahydro-cyclopenta[b]furane, ni la 3-méthyl-3-(6,6,6a-triméthyl-hexahydro-cyclopenta[b]furan-2-yl)-butanone.

2. Composé selon la revendication 1, dans lequel R¹, R², R³ et R⁴ sont, indépendamment, un atome d'hydrogène ou un groupe méthyle, éthyle, n-propyle, *i-*propyle, *n*-butyle, *i*-butyle, *s*-butyle, *t*-butyle, *n-*pentyle, *i*-pentyle, *t*-amyle, 1-éthyl-propyle, 1,2-diméthyl-propyle, 2,2-diméthyl-propyle, *n*-hexyle, 1-méthyl-pentyle, un groupe 2-méthyl-pentyle, un groupe 3-méthyl-pentyle, un groupe 4-méthyl-pentyle, 1,1-diméthyl-butyle, 1,2-diméthyl-butyle, 1,3-diméthyl-butyle, 2,2-diméthyl-butyle, 3,3-diméthyl-butyle, 1-éthyl-butyle, 2-éthyl-butyle, 1,1,2-triméthyl-propyle, 1,2,2-triméthyl-propyle, 1-éthyl-1-méthyl-propyle ou 1-éthyl-2-méthyl-propyle facultativement substitué, ou un groupe vinyle, 1-propényl, 1-*i*-propényle, 2-propényle, 2-méthyl-1-propényle, 1-butényle, 2-butényle, 3-butényle, 1-pentényle, 2-pentényle, 1-hexényle, 2-hexényle, 3-hexényle, 2,4-pentadiényl facultativement substitué, à condition que le substituant facultatif ne soit pas un groupe phényle.

3. Composé selon la revendication 1 ou 2, dans lequel R¹, R² et R³ sont chacun un atome d'hydrogène et R⁴ est un groupe alkyle en C₁ à C₆ ou alcényle en C₂ à C₆ linéaire ou ramifié substitué, à condition que le substituant facultatif ne soit pas un groupe phényle.

4. Composé de formule (I), choisi dans le groupe consistant en le 2-isobutyl-6,6,6a-triméthyl-hexahydro-cyclopenta[b]furane, le 2-*sec*-butyl-6,6,6a-triméthyl-hexahydro-cyclopenta[b]furane, le 2-butyl-6,6,6a-triméthyl-hexahydro-cyclopenta[b]furane, le 6,6,6a-triméthyl-2-vinyl-hexahydro-cyclopenta[b]furane.

5. Procédé de préparation d'un composé de formule générale (I) : 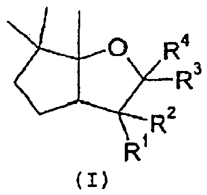 dans laquelle
R¹, R², R³ et R⁴ sont, indépendamment, un atome d'hydrogène ou un groupe alkyle en C₁ à C₆ ou alcényle en C₂ à C₆ linéaire ou ramifié facultativement substitué,
le procédé comprenant la réaction d'un composé de formule (III) : 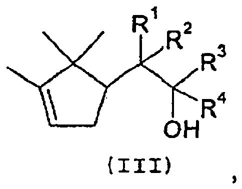 dans laquelle
R¹, R², R³ et R⁴ sont définis comme en rapport avec la formule (I),
avec de l'acide phosphorique dans un solvant organique de façon à obtenir un mélange comprenant un composé de formule (I) et un composé de formule (II) : 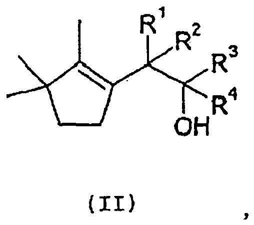 dans laquelle
R¹, R², R³ et R⁴ sont définis comme en rapport avec la formule (I).

6. Procédé selon la revendication 5, dans lequel le solvant organique est choisi dans le groupe comprenant le toluène, le xylène, le triméthylbenzène, le cyclohexane et le méthylcyclohexane.

7. Procédé selon la revendication 5 ou 6, dans lequel l'acide phosphorique est utilisé dans une quantité de 1 à 10 % en poids du composé (III).

8. Procédé selon l'une quelconque des revendications 5 à 7, comprenant en outre la réaction du composé de formule (II) avec de l'acide trifluorométhanesulfonique.

9. Procédé selon la revendication 8, comprenant, avant la réaction avec de l'acide trifluorométhanesulfonique, la séparation et la purification du composé de formule (II).

10. Procédé selon la revendication 9, dans lequel l'acide trifluorométhanesulfonique est utilisé dans une quantité de 0,5 à 2 % en poids du composé (II).

11. Composé de formule générale (II) : 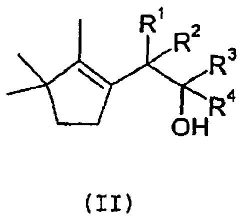 dans laquelle
R¹, R², R³ et R⁴ sont, indépendamment, un atome d'hydrogène ou un groupe alkyle en C₁ à C₆ ou alcényle en C₂ à C₆ linéaire ou ramifié facultativement substitué,
à condition que lorsque chacun de R¹, R² et R³ est un atome d'hydrogène, R⁴ ne soit pas un groupe alkyle en C₁ à C₆ ou alcényle en C₂ à C₆ linéaire ou ramifié substitué par phényle ; et
à condition que le composé de formule (II) ne soit ni le 2-(2,3,3-triméthyl-cyclopent-1-ényl)-éthanol, le 1-(2,3,3-triméthyl-cyclopent-1-ényl)-butan-2-ol, ni le 1-(2,3,3-triméthyl-cyclopent-1-ényl)-pent-3-én-2-ol.

12. Composition parfumée contenant au moins un composé selon l'une quelconque des revendications 1 à 4 et 11.

13. Utilisation d'un composé de formule (I) ou de formule (II) : 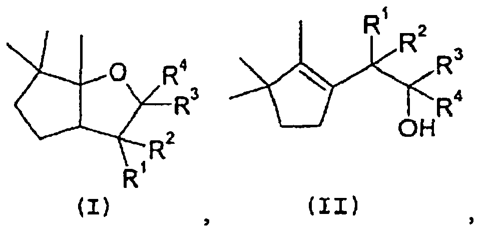 dans lesquelles R¹, R², R³ et R⁴, sont, indépendamment, un atome d'hydrogène ou un groupe alkyle en C₁ à C₆ ou alcényle en C₂ à C₆ linéaire ou ramifié facultativement substitué, ou d'une composition selon la revendication 12, dans le domaine de la parfumerie, pour la préparation de bases et concentrés parfumés, de fragrances, de parfums ; de compositions topiques ; de compositions cosmétiques, telles que des crèmes pour le visage et le corps, des nettoyants, un soin du visage, des poudres à base de talc, des huiles capillaires, des shampooings, des lotions pour les cheveux, des huiles et sels pour le bain, des gels et bains douches, des savons, des antitranspirants et déodorants corporels, des crèmes et lotions de prérasage, rasage et après-rasage, des crèmes, des dentifrices, des bains de bouche et des pommades ; et des produits nettoyants, tels que des adoucissants, des détergents, des désodorisants et des produits d'entretien ménagers.

14. Utilisation d'un composé de formule (I) ou de formule (II) : 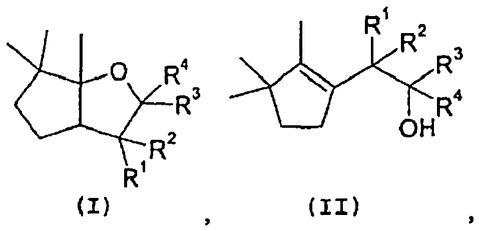 dans lesquelles R¹, R², R³ et R⁴, sont, indépendamment, un atome d'hydrogène ou un groupe alkyle en C₁ à C₆ ou alcényle en C₂ à C₆ linéaire ou ramifié facultativement substitué, ou d'une composition selon la revendication 12, comme agent masquant les odeurs.
